# EUROPEAN PATENT SPECIFICATION

(11) **EP 1 304 991 B1**
(45) Date of publication and mention of the grant of the patent: **03.09.2014**
(21) Application number: 01952509.6
(22) Date of filing: 06.07.2001
(51) Int. Cl.: A61K 9/08, A61P 27/04

(54) **METHOD AND KIT FOR MOISTURIZING THE SURFACE OF THE EYE**
METHODE UND KIT FÜR DIE BEFEUCHTUNG DER AUGENOBERFLÄCHE
PROCEDE ET KIT D'HYDRATATION DE LA SURFACE DE L'OEIL

(30) Priority: 12.07.2000 US 614790; 30.03.2001 US 823385
(43) Date of publication of application: 02.05.2003
(73) Proprietor: Rogue Valley Natural Springs, Inc., Grants Pass, OR 97526 (US)
(72) Inventor: KLEYNE, Sharon, F., Grants Pass, OR 97526 (US)
(74) Representative: Barton, Matthew Thomas
(86) International application number: PCT/US2001/021490
(87) International publication number: WO 2002/003924

(56) References cited:
- WO-A1-96/00050
- WO-A1-97/23177
- US-A- 4 753 945
- US-A- 5 032 392
- US-A- 5 294 607
- US-A- 5 997 518

## Description

### Field of the Invention

The invention pertains to the field of care of the surface of the eye, including the sclera, conjunctiva, and cornea. More particularly, the invention pertains to the application of fluids for moisturizing the surface of the eye.

### BACKGROUND OF THE INVENTION

In normal situations, the surface of the eye, including the sclera, the conjunctiva, and the cornea, is kept moist by the presence of a tear film. This tear film is found in virtually all terrestrial vertebrates, with the exception of snakes.

The surface area of the eye in an adult human is about 2 cm². It is covered by a complex tear film having a trilaminar structure, with each of the layers having a discrete and necessary function.

Nearest to the surface of the eye is an inner layer of mucus approximately 10 to 20 µm in thickness. The mucus in this layer stabilizes the tear film and provides for attachment of the tear film to the underlying cornea and conjunctiva. The mucus also reduces the surface tension between the tear film and the eye and so permits the tear film to spread evenly across the eye.

The middle layer of the eye is an aqueous layer that is composed largely of water, electrolytes, and various proteins. This layer contains about 2 to 5 µl of aqueous fluid and forms the bulk of the tear film. Within this layer, pH, osmotic pressure, oxygen tension, and the levels of electrolytes such as potassium, calcium, chloride, inorganic phosphates, and acids such as lactic acid and citric acid, are maintained within narrow physiologic ranges. Proteins present in the aqueous layer of the tear film include albumin, and other proteins, such as immunoglobulins, interferon, β-lysin, and lysozyme which have antimicrobial activities.

Farthest from the surface of the eye is a lipid layer, which may range in thickness from a single monolayer to nearly 200 nm. Ordinarily, this layer is about 100 nm thick. This layer serves to retard evaporation of the tear film.

The tear film rapidly decreases in thickness following a blink. Without a subsequent blink, holes will begin to form in the tear film, called tear breakup, within about 30 seconds. Tear breakup times lower than 10 seconds are considered to be abnormal. This can occur with decreased tear formation or deficiencies in the mucus layer of the tear film. Other situations that can result in dryness of the eye surface include environmental aridity, contact lens wearing, and upon waking.

Typically, dryness of the eye is treated with water based solutions containing electrolytes and preservatives which maintain sterility of the solution for multiple applications. Solutions without preservatives are usually packaged in containers that provide for a single use, with disposal of the container and any residual solution following the single application.

The solutions are generally applied by drops, which provide about 20 to 25 µl of fluid to the eye surface. The application of eye drops results in rapid moisturizing of the eye. However, because the amount delivered is greater than the volume of the tear film, these drops have the disadvantage of flooding the eye, which washes away the tear film and replaces the tear film with the fluid that comprises the drops. Immediately following this flooding there exists a period of time when the normal tear film, with its three layer structure and the constituents of each layer, is not present on the eye surface. This can result in incomplete eye moisturizing which lasts for several blink cycles.

Other methods of administration of liquids onto the surface of the eye include eye cups, aerosol and pump sprays, and misters. Eye cups are used to bathe the surface of the eye in fluid, which results in flooding and washing away the tear film that is present on the eye surface. A mister that can be used to deliver a spray of droplets to the eye is described in Hahn, U.S. Patents Nos. 5,346,132 and 5,893,515.

In these patents, Hahn discloses several disadvantages of delivering fluid to the eye by drops, including difficulty in positioning the dropper and incomplete delivery of medications due to missing the eye and spilling onto the face. Hahn does not address the issue of the quantity of fluid that is administered to the eye or the issue of washing away the tear film due to flooding. The mister of Hahn delivers a measurable quantity of fluid and can be used for household or medical purposes or to moisturize the eyes or the skin.

Another mister is described in Hutson, U.S. Patent No. 5,588,564. Like the mister of Hahn, the mister of Hutson can be used to deliver an adjustable and repeatable dose of fluid to the surface of the eye. Hutson does not address the issue of the quantity of fluid that is administered to the eye or the issue of washing away the tear film due to flooding. WO97/23177 discloses the dosage of ophthalmic treatment liquid in the form of a jet or stream of droplets.

A need exists for a method to moisturize the surface of the eye without flooding the eye or destroying the integrity of the natural tear film.

### BRIEF SUMMARY OF THE INVENTION

It has been unexpectedly discovered that administering an amount of fluid to the surface of the eye at a level below that which results in flooding and washing away the tear film results in an improvement in eye moisturizing over prior art methods.

In one embodiment, the invention is a method for moisturizing the eye. The method comprises steps as disclosed in the claims. The method according to the disclosure includes obtaining an applicator that can controllably deliver an aqueous fluid to the surface of the eye in a quantity below that which will flood the eye. In this manner, the method of the disclosure serves to rehydrate the aqueous layer of the tear film and leaves the normal trilaminar tear film intact. In accordance with the method of the invention, the quantity of fluid that is administered to the eye surface is less than about two times the volume of the normal aqueous layer of the tear film, that is less than about 10 µl. Preferably, between 0.5 and 6 µl is administered, and most preferably, between 2 and 5 µl is administered. The fluid may be administered to the eye surface in a single bolus, or may be administered over time, in ten seconds or less, preferably 5 seconds or less, in accordance with the invention.

The fluid is delivered as a fine mist. It has been discovered that aqueous fluids in the form of a fine mist are extremely well suited for rehydrating the aqueous portion of the tear film, without rinsing away the tear film.

In another embodiment, the invention is a kit for delivering a composition for moisturizing the eye. In accordance with the invention, the kit contains an aqueous fluid composition, a container that holds the composition, and an applicator that, when actuated, controllably administers between about 0.5 and 10 µl of the pharmaceutical composition to a surface of about 2 cm² in about 10 seconds or less, preferably about 5 seconds or less. Preferably, the kit further contains instructions to controllably apply the pharmaceutical composition to the surface of the eye using the kit.

According to the present disclosure there is provided the use of an aqueous fluid consisting essentially of water for the manufacture of a product for moisturizing the eye, wherein the aqueous fluid is for spraying onto the surface of the eye in the form of a mist consisting essentially of droplets having an average diameter between 5 and 150 microns and wherein the amount of the aqueous fluid that is for spraying onto the surface of the eye is sufficient to hydrate the aqueous layer of the tear film of the eye but is below that which will wash away the tear film.

In a further aspect of the present invention, there is provided an eye-moisturizing kit comprising a sealed container, an aqueous fluid consisting essentially of water within said container, and an actuator that sprays a mist of said fluid from said container onto the surface of the eye in an amount that is sufficient to moisturize the aqueous layer of the tear film on the surface of the eye and less than an amount that washes away the tear film.

The present invention is set out in the appended dependent claims.

### BRIEF DESCRIPTION OF THE DRAWING

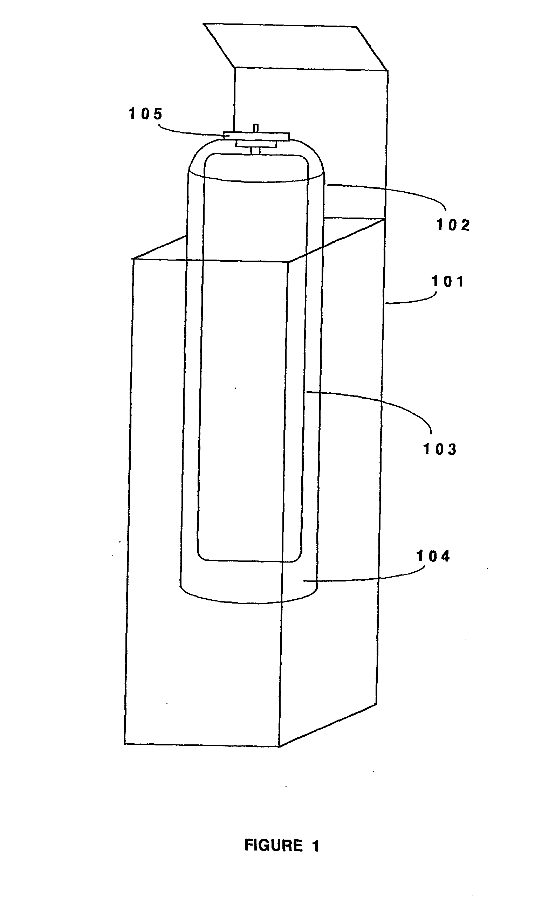

FIG. 1 shows one embodiment of the kit of the invention for moisturizing the eye in accordance with the method of the invention.

### DETAILED DESCRIPTION OF THE INVENTION

It has been discovered that a sudden increase in humidity to the tear film, as opposed to a splash of fluid such as occurs with presently available droppers and misters, increases the water content of the tear film while causing little or no displacement of the tear film.

In accordance with the invention, the volume of the tear film the surface of the eye is increased by applying a fluid in an amount not greater than about 100 to 200% of the volume of the aqueous portion of the tear film, which generally has a volume of 2 to 5 µl. Thus, in accordance with the invention, about 10 µl or less is applied to the surface of the eye. Preferably, 0.5 to 6 µl is applied, and most preferably 1 to 2 µl is applied, especially when moisturizing the eye because of the presence of dry eye, in which the total tear volume is typically between 1 to 2 µl. The volume of fluid in accordance with the invention acts to rehydrate the aqueous portion of the tear film and maintains the integrity of the overlying lipid and the underlying mucus layers.

In contrast with the present state of the art in which 20 to 50 µl of fluid is applied to the eye by dropper or by spray, the method in accordance with the invention reestablishes the normal state in individuals with dry eye. Present methods merely wash away the existing tear film and replace the tear film, or at least the middle aqueous layer, with an aqueous solution. These solutions lack the structure of the intact tear film and also differs from the normal aqueous layer of the tear film.

Generally, the above amount of fluid which is applied in accordance with the invention is applied during one blink cycle, that is between blinks. However, the fluid may be applied during a period of time in which one or more blinks occurs. Preferably, the fluid is applied within a period of 5 to 10 seconds or less.

The fluid is administered as dispersed droplets in air (mist), in particular in the form of a fine mist of discrete liquid droplets in which the average size of the fluid droplets is between about 5 and 150 microns in diameter. It has been found that a fine mist composed of droplets of this size range, preferably between about 0.1 % to 1 % of the tear volume per droplet, provides optimal hydration of the tear film and moisturization of the surface of the eye. Preferably, the average size of the fluid droplets is less than 100 microns, and more preferably less than 75 microns. Most preferably, the droplets have a diameter between 10 and 50 microns, with a most preferred range between 15 and 30 microns in diameter. Droplets above about 100 microns in diameter tend to incompletely vaporize and will fall out and produce undesirable wetting of the face and on horizontal surfaces. Droplets below about 20 microns in diameter are generally considered to be inhalable and can be aspirated into the upper and lower respiratory passages. This is acceptable when delivering a substance to the surface of the eye which is not potentially harmful to the respiratory system, such as a water.

The fluid that is delivered to the surface of the eye in accordance with the invention is a water based fluid. For moisturizing the eye, the fluid preferably is an aqueous fluid having a pH of neutral to slightly acidic, such as between about 7 to about 6.5. Preferably, the fluid has a low concentration of solutes, less than that of the normal tear film. In accordance with a preferred embodiment of the invention, the osmotic pressure of the fluid is less than 350 mOsm and most preferably less than 311 mOsm.

It is further preferred that the water be hypoallergenic and substantially free of preservatives and other chemical compounds that have a potential to irritate the surface of the eye.

The aqueous fluid delivered to the surface of the eye consists essentially of water. The water may contain trace amounts of minerals. It is most preferred, however, that the amount of minerals in the fluid be as small as possible. The water is free of constituents such as lubricants, preservatives, buffering agents and surfactants.

In another embodiment, the invention is a kit for administering a controlled dosage of between 0.5 and less than 10 µl and most preferably less than 5 µl. In the most preferred embodiment, the controlled dosage is between 1 and 2 µl of fluid. In accordance with the invention, the kit contains a container, an aqueous fluid within the container, and an actuator that delivers a spray or fine mist of fluid in the dosage described above. It is preferred that the mist be composed of discrete droplets having an average size of about 5 to 150 microns in diameter, most preferably less than 100 microns, even more preferably less than 75 microns, most preferably less between 10 and 50 microns with a most preferred range between 15 and 30 microns in diameter. The kit may further contain instructions to apply the controlled dosage of the aqueous fluid to the surface of the eye. Preferably, the container of the kit is hermetically sealed so that it may be used for multiple applications of the aqueous fluid over several days to months without the need to include a preservative in the fluid.

A preferred embodiment of the kit of the invention is shown diagrammatically in FIG. 1. FIG. 1 shows a package such as a box 101 for containing a rigid, preferably metallic, hermetically sealed container 102, inside of which is an inner hermetically sealed flexible pouch 103, which contains a fluid to be dispensed. There is a pressurization agent such as compressed air or nitrogen 104 between the hermetically sealed container 102 and the flexible pouch 103, and an actuator 105 that permits the proper dosage of the fluid in the pouch 103 to escape when depressed. The kit further contains instructions (not shown) for delivering a pre-determined dosage of the fluid into the eye.

The invention is further described in the following non-limiting examples.

### Example 1

The volume of the tear film on the eyes of three adult human subjects is measured and determined to average 2.26 µl. The subjects are then treated by administering to surface of their eyes fine mist of between 50 and 100 micron average droplet size, with a total volume of between 2 to 5 µl within a period of 10 seconds per eye. Following administration, the tear volume is again measured and is determined to average 2.96 µl.

### Example 2

Samples of the tear film from three adult human subjects are obtained and subjected to HPLC chromatography to determine the baseline level of proteins and other constituents in the tear film. One eye from each of the subjects is then moisturized by administration of a standard drop of artificial tears of between 25 and 50 µl. Samples of the tear film from the three treated eyes are then obtained and subjected to HPLC chromatography. After obtaining the second group of samples, the opposite eye of each of the subjects is then moisturized by administration of the same artificial tears but in a fine mist made of droplets having a size between 50 and 100 microns for a total volume of about 5 µl. Samples of the tear film from the mist-treated eyes are then obtained and subjected to HPLC chromatography. The artificial tears are also subjected to HPLC chromatography.

The initial HPLC chromatography provides a profile of the constituents found in the normal tear film. It is found to contain various lipids, mucus, proteins, and electrolytes.

The HPLC chromatography following moisturization by a single large drop reveals that most if not all of the lipids and mucus remain in the tear film. The proteins and electrolytes that are present in the normal tear film are no longer present and the tear film has a chromatography profile similar to that of the artificial tears, minus the lipids and mucus.

The HPLC chromatography following moisturization by the fine mist reveals that the lipids and mucus remain in the tear film. The proteins and electrolytes that are present in the normal tear film are demonstrated by the chromatography to remain in the tear film following moisturization by the fine mist.

## Claims

1. A non-therapeutic method for moisturizing the eye, the method comprising delivering an aqueous fluid consisting of water and, optionally, trace amounts of minerals, onto the surface of the eye in the form of a mist consisting of dispersed aqueous fluid droplets in air having an average diameter of between 5 and 150 µm (microns), wherein the amount of the aqueous fluid is between 0.5 and 10µl.

2. The method according to Claim 1, wherein the aqueous fluid has an osmolarity of less than that of the normal aqueous layer of the tear film. ,

3. The method according to any of Claims 1 and 2, wherein the osmolarity of the aqueous fluid is less than 311 mOsm.

4. The method according to any of Claims 1 to 3, wherein the pH of the aqueous fluid is between 7 and 6.5.

5. The method according to Claim 4, wherein the pH is 6.5.

6. The method according to any of Claims 1 to 5, wherein the aqueous fluid is delivered within a period of 5 seconds.

7. The method according to any of Claims 1 to 6, wherein between 0.5 and 10 µl is delivered onto the surface of the eye.

8. The method according to Claim 7, wherein between 0.5 and 6 µl is delivered onto the surface of the eye.

9. The method according to Claim 8, wherein between 2 and 5 µl is delivered onto the surface of the eye.

10. The method according to any preceding claim, wherein the mist is delivered from a device comprising a sealed container, an aqueous fluid consisting of water and, optionally, trace amounts of minerals, within said container, and an actuator for delivering a mist of the aqueous fluid from said container, and wherein the aqueous fluid is delivered to the surface of the eye within a period of 5 seconds or less.

11. The method according to Claim 10, wherein the device further comprises a sealed flexible pouch containing said aqueous fluid within said container and a pressurization agent between said container and said pouch.

12. An eye-moisturizing kit comprising a sealed container, an aqueous fluid consisting of water and, optionally, trace amounts of minerals, within said container, and an actuator that sprays a mist of said aqueous fluid from said container onto the surface of the eye, said mist consisting of dispersed aqueous fluid droplets in air having an average diameter between 5 and 150 µm (microns) in a controlled dosage of between 0.5 and 10 µl.

13. The kit of Claim 12 which further comprises a sealed flexible pouch within said container, which pouch contains said aqueous fluid, and a pressurization agent between said container and said pouch.

14. The kit of Claims 12 or 13, wherein the actuator controllably administers between 0.5 and 10 µl of the aqueous fluid to a surface of 2 cm² in 5 seconds or less.

15. The kit of any of Claims 12 to 14 which further comprises a package housing said sealed container and instructions for applying the aqueous fluid to the surface of the eye.

16. The kit of any one of Claims 12 to 15, wherein the mist consists of droplets of the aqueous fluid having an average diameter of less than 100 microns.

17. The kit of any of Claims 12 to 16, wherein the container is a rigid container.

18. The kit of Claim 17, wherein the rigid container is metallic.

## Patentansprüche

1. Nicht therapeutisches Verfahren für die Befeuchtung des Auges, wobei das Verfahren umfasst:
Lieferung einer wässrigen Flüssigkeit, die aus Wasser und, optional, Spurenmengen von Mineralen besteht, auf die Oberfläche des Auges in Form eines Nebels, der aus dispergierten wässrigen Flüssigkeitströpfchen in Luft besteht, die einen durchschnittlichen Durchmesser zwischen 5 und 150 µm (Mikron) aufweisen, wobei die Menge der wässrigen Flüssigkeit zwischen 0,5 und 10 µl beträgt.

2. Verfahren nach Anspruch 1, wobei die wässrige Flüssigkeit eine Osmolalität von weniger als die der normalen wässrigen Schicht des Tränenfilms aufweist.

3. Verfahren nach einem beliebigen der Ansprüche 1 und 2, wobei die Osmilalität der wässrigen Flüssigkeit weniger als 311 mOsm beträgt.

4. Verfahren nach einem beliebigen der Ansprüche 1 bis 3, wobei der pH-Wert der wässrigen Flüssigkeit zwischen 7 und 6,5 beträgt.

5. Verfahren nach Anspruch 4, wobei der pH-Wert 6,5 beträgt.

6. Verfahren nach einem beliebigen der Ansprüche 1 bis 5, wobei die wässrige Flüssigkeit innerhalb eines Zeitraums von 5 Sekunden zugeführt wird.

7. Verfahren nach einem beliebigen der Ansprüche 1 bis 6, wobei zwischen 0,5 und 10 µl auf die Augenoberfläche geliefert werden.

8. Verfahren nach Anspruch 7, wobei zwischen 0,5 und 6 µl auf die Augenoberfläche geliefert werden.

9. Verfahren nach Anspruch 8, wobei zwischen 2 und 5 µl auf die Augenoberfläche geliefert werden.

10. Verfahren nach einem beliebigen vorangehenden Anspruch, wobei der Nebel aus einer Vorrichtung geliefert wird, die einen abgedichteten Behälter, eine wässrige Flüssigkeit, die aus Wasser und, optional, Spurenmengen von Mineralen, innerhalb des besagten Behälters besteht, und einen Aktuator zur Lieferung eins Nebels der wässrigen Flüssigkeit aus dem Behälter umfasst und, wobei die wässrige Flüssigkeit innerhalb eines Zeitraums von weniger als 5 Sekunden oder weniger auf die Augenoberfläche geliefert wird.

11. Verfahren nach Anspruch 10, wobei die Vorrichtung ferner einen abgedichteten, flexiblen Beutel, der die besagte wässrige Flüssigkeit innerhalb des besagten Behälters enthält und ein Druckmittel zwischen dem besagten Behälter und dem besagten Beutel umfasst.

12. Kit für die Befeuchtung der Augenoberfläche, der einen abgedichteten Behälter, eine wässrige Flüssigkeit, die aus Wasser und, optional, Spurenmengen von Mineralen, innerhalb des besagten Behälters, besteht, und einen Aktuator umfasst, der einen Nebel der besagten wässrigen Flüssigkeit aus dem besagten Behälter in einer kontrollierten Dosierung zwischen 0,5 und 10 µl auf die Augenoberfläche sprüht, wobei der besagte Nebel aus dispergierten wässrigen Flüssigkeitströpfchen in Luft besteht, die einen durchschnittlichen Durchmesser zwischen 5 und 150 µm (Mikron) aufweisen.

13. Kit nach Anspruch 12, der ferner einen abgedichteten, flexiblen Beutel innerhalb des besagten Behälters, wobei der Beutel die besagte wässrige Flüssigkeit enthält, und ein Druckmittel zwischen dem besagten Behälter und dem besagten Beutel umfasst.

14. Kit nach Ansprüchen 12 oder 13, wobei der Aktuator steuerbar zwischen 0,5 und 10 µl der wässrigen Flüssigkeit in 5 Sekunden oder weniger auf eine Oberfläche von 2 cm² verabreicht.

15. Kit nach einem beliebigen der Ansprüche 12 bis 14, der ferner eine Packung, in welcher der besagte abgedichtete Behälter untergebracht ist, und Anweisungen zum Aufbringen der wässrigen Flüssigkeit auf die Augenoberfläche umfasst.

16. Kit nach einem beliebigen der Ansprüche 12 bis 15, wobei der Nebel aus Tröpfchen der wässrigen Flüssigkeit besteht, die einen durchschnittlichen Durchmesser von weniger als 100 Mikron aufweisen.

17. Kit nach einem beliebigen der Ansprüche 12 bis 16, wobei der Behälter ein starrer Behälter ist.

18. Kit nach Anspruch 17, wobei der starre Behälter metallisch ist.

## Revendications

1. Procédé non thérapeutique d'hydratation de l'oeil, le procédé consistant à administrer un fluide aqueux constitué d'eau et, éventuellement, de minéraux à l'état de traces, sur la surface de l'oeil sous la forme d'un brouillard consistant en des gouttes de fluide aqueux dispersées dans l'air, présentant un diamètre moyen compris entre 5 et 150 µm (microns), la quantité de fluide aqueux étant comprise entre 0,5 et 10 µl.

2. Procédé selon la revendication 1, dans lequel le fluide aqueux présente une osmolalité inférieure à celle de la couche aqueuse normale du film lacrymal.

3. Procédé selon l'une quelconque des revendications 1 et 2, dans lequel l'osmolalité du fluide aqueux est inférieure à 311 mOsm.

4. Procédé selon l'une quelconque des revendications 1 à 3, dans lequel le pH du fluide aqueux est compris entre 7 et 6,5.

5. Procédé selon la revendication 4, dans lequel le pH est égal à 6,5.

6. Procédé selon l'une quelconque des revendications 1 à 5, dans lequel le fluide aqueux est administré en une période de 5 secondes.

7. Procédé selon l'une quelconque des revendications 1 à 6, dans lequel une quantité comprise entre 0,5 et 10 µl est administrée sur la surface de l'oeil.

8. Procédé selon la revendication 7, dans lequel une quantité comprise entre 0,5 et 6 µl est administrée sur la surface de l'oeil.

9. Procédé selon la revendication 8, dans lequel une quantité comprise entre 2 et 5 µl est administrée sur la surface de l'oeil.

10. Procédé selon l'une quelconque des revendications précédentes, dans lequel le brouillard est administré par un dispositif comprenant un récipient étanche, un fluide aqueux constitué d'eau et, éventuellement, de minéraux à l'état de trace, dans ledit récipient, et un actionneur pour administrer un brouillard du fluide aqueux contenu dans ledit récipient, et dans lequel le fluide aqueux est administré sur la surface de l'oeil en une période 5 secondes ou moins.

11. Procédé selon la revendication 10, dans lequel le dispositif comprend en outre une poche souple étanche contenant ledit fluide aqueux dans ledit récipient, et un agent de pressurisation entre ledit récipient et ladite poche.

12. Kit d'hydratation de l'oeil comprenant un récipient étanche, un fluide aqueux constitué d'eau et, éventuellement, de minéraux à l'état de traces, dans ledit récipient, et un actionneur qui pulvérise sur la surface de l'oeil un brouillard dudit fluide aqueux contenu dans ledit récipient, ledit brouillard consistant en des gouttes de fluide aqueux dispersées dans l'air, présentant un diamètre moyen compris entre 5 et 150 µm (microns) dans une dose contrôlée comprise entre 0,5 et 10 µl.

13. Kit selon la revendication 12, comprenant en outre une poche souple étanche dans ledit récipient, ladite poche contenant ledit fluide aqueux, et un agent de pressurisation entre ledit récipient et ladite poche.

14. Kit selon la revendication 12 ou 13, dans lequel l'actionneur administre de façon contrôlée une quantité comprise entre 0,5 et 10 µl du fluide aqueux sur une surface de 2 cm2 en 5 secondes ou moins.

15. Kit selon l'une quelconque des revendications 12 à 14, comprenant en outre un emballage contenant ledit récipient étanche et des instructions pour l'application du fluide aqueux sur la surface de l'oeil.

16. Kit selon l'une quelconque des revendications 12 à 15, dans lequel le brouillard consiste en des gouttes du fluide aqueux présentant un diamètre moyen inférieur à 100 microns.

17. Procédé selon l'une quelconque des revendications 12 à 16, dans lequel le récipient est un récipient rigide.

18. Kit selon la revendication 17, dans lequel le récipient rigide est métallique.
